# EUROPÄISCHE PATENTSCHRIFT

(11) **EP 3 069 719 B1**
(45) Veröffentlichungstag und Bekanntmachung des Hinweises auf die Patenterteilung: **09.10.2019**
(21) Anmeldenummer: 15159905.7
(22) Anmeldetag: 19.03.2015
(51) Int. Cl.: A61K 31/4375, A61P 9/12

(54) **RENALE DENERVIERUNG**
RENAL DENERVATION
DÉNERVATION RÉNALE

(43) Veröffentlichungstag der Anmeldung: 21.09.2016
(73) Patentinhaber: B. Braun Melsungen AG, 34212 Melsungen (DE)
(72) Erfinder: Calisse, Jorge, 12203 Berlin (DE); Boxberger, Michael, 12103 Berlin (DE); Stöver, Michael, 12439 Berlin (DE); Stolle, Andreas, 12101 Berlin (DE); Kaiser, Martin, 06179 Teutschenthal (DE); Berg, Madeleine, 12109 Berlin (DE)
(74) Vertreter: Patentanwälte Ruff, Wilhelm, Beier, Dauster & Partner mbB

(56) Entgegenhaltungen:
- WO-A1-2010/124120
- WO-A1-2013/059735
- WO-A1-2013/188689
- US-A1- 2011 182 912
- US-B1- 6 178 349
- E. MEILMAN ET AL: "Clinical Studies on Veratrum Alkaloids: I. The Action of Protoveratrine and Veratridine in Hypertension", CIRCULATION, Bd. 1, Nr. 2, 1. Februar 1950 (1950-02-01) , Seiten 204-213, XP055205011, ISSN: 0009-7322, DOI: 10.1161/01.CIR.1.2.204
- PAUL M. CONSIGNY ET AL: "Chemical Renal Denervation in the Rat", CARDIOVASCULAR AND INTERVENTIONAL RADIOLOGY, Bd. 37, Nr. 1, 3. Dezember 2013 (2013-12-03), Seiten 218-223, XP055204988, ISSN: 0174-1551, DOI: 10.1007/s00270-013-0796-7
- BARBASH ISRAEL M ET AL: "Sympathetic renal denervation: Hypertension beyond SYMPLICITY", CARDIOVASCULAR REVASCULARIZATION MEDICINE, Bd. 14, Nr. 4, 2013, Seiten 229-235, XP028689201, ISSN: 1553-8389, DOI: 10.1016/J.CARREV.2013.02.004

## Beschreibung

Die Erfindung betrifft eine neurotoxische Substanz zur Anwendung bei der Behandlung und/oder Vorbeugung von Bluthochdruck und/oder Krankheiten, die im Zusammenhang mit Bluthochdruck stehen, sowie ein medizinisches Kit.

Die US 2011/0182912 A1 offenbart verschiedene Wirkstoffe, welche für eine Verabreichung in die renale Arterienwand vorgesehen sein können sowie deren Einfluss auf das Neuralgewebe. Als Wirkstoff wird unter anderem Veratridin genannt.

Nach der Global Burden of Disease Study 2010 ist Bluthochdruck gegenwärtig eine der großen Gesundheitsgefahren. Mögliche (letale) Folgen sind Schlaganfall und Herzinfarkt.

Unter Bluthochdruck versteht man im Allgemeinen ein Krankheitsbild, bei dem der Blutdruck des arteriellen Gefäßsystems chronisch erhöht ist. Nach der Definition der Weltgesundheitsorganisation (WHO) gilt ein systolischer Blutdruck von zumindest 140 mm Hg oder ein diastolischer Blutdruck von zumindest 90 mm Hg als Hypertonie (bzw. arterielle Hypertonie).

Man unterscheidet zwei Formen von Bluthochdruck. Bei der sogenannten primären oder essentiellen Hypertonie, welche bei der überwiegenden Mehrzahl der Bluthochdruckpatienten (Hypertoniker) vorliegt, können keine körperlichen Ursachen festgestellt werden. Die primäre Hypertonie muss in vielen Fällen langfristig oder lebenslang mit blutdrucksenkenden Medikamenten behandelt werden. Hinzu kommt, dass 35 % der Hypertoniker nicht auf etablierte medikamentöse Behandlungsmöglichkeiten ansprechen, d.h. unter einer sogenannten therapierefraktären Hypertonie leiden.

Bei der sogenannten sekundären Hypertonie kann eine zugrundeliegende Erkrankung identifiziert werden. Eine sekundäre Hypertonie wird behandelt, indem die zugrundeliegende Erkrankung therapiert wird.

Bei der Regulierung des Bluthochdrucks ist die Niere von zentraler Bedeutung. Bei einer erhöhten efferenten Aktivität des Sympathikus und der daraus erhöhten Nierenaktivität werden die blutdruckerhöhenden Stoffe Renin und Noradrenalin freigesetzt.

Seit 2008 steht mit der sogenannten renalen Denervierung (renale Denervation) ein Verfahren zur Behandlung von Bluthochdruck zur Verfügung, bei welchem Nervenbahnen zwischen dem Gehirn und der Niere unterbrochen werden. Die renale Denervierung beruht auf einer Schädigung, insbesondere Ablation, von Nervenfasern des sympathischen Nervensystems, die sich in den Gefäßwänden der Nierenarterien, insbesondere in der äußeren Gefäßwandschicht Tunica adventitia, befinden (renaler Sympathikus). Die renalen Nervenfasern können mittels Abgabe von Energie, beispielsweise HF-Energie (Hochfrequenz-Energie) über Elektroden, die mittels eines Katheters in die beiden Nierenarterien (Arteria renalis sinistra und Arteria renalis dextra) vorgeschoben werden, oder mittels Injektion von Ethanol oder Guanethidin geschädigt, insbesondere ablatiert, werden.

Die renale Denervierung bietet sich insbesondere als alternative Behandlungsmöglichkeit bei Patienten an, die an einer therapierefraktären Hypertonie leiden. Nachteilig ist indes, dass die bislang zur Verfügung stehenden Möglichkeiten zur renalen Denervierung nicht nur eine Schädigung der renalen Nervenfasern, sondern auch des umliegenden Körpergewebes bewirken. Im Falle einer Denervierung mittels Guanethidin besteht die Schädigung der Nervenfasern obendrein nur in einer vorübergehenden Beeinträchtigung der renalen Nervenfunktion. Eine persistente Denervierung ist nicht möglich.

### Aufgabe und Lösung

Es ist eine Aufgabe der Erfindung, eine neurotoxische Substanz bereitzustellen, die eine Behandlung und/oder Vorbeugung von Bluthochdruck und/oder Krankheiten, die im Zusammenhang mit Bluthochdruck stehen, ermöglicht. Es ist des Weiteren eine Aufgabe der Erfindung, ein medizinisches Kit bereitzustellen.

Diese Aufgaben werden gelöst durch eine neurotoxische Substanz gemäß dem unabhängigen Anspruch 1 sowie ein medizinisches Kit gemäß Anspruch 8. Bevorzugte Ausführungsformen sind in den abhängigen Ansprüchen 2 bis 7 definiert. Der Wortlaut sämtlicher Ansprüche wird hiermit durch ausdrückliche Bezugnahme zum Inhalt der vorliegenden Beschreibung gemacht.

Gemäß einem ersten Aspekt betrifft die Erfindung eine neurotoxische Substanz zur Anwendung bei der Behandlung und/oder Vorbeugung von Bluthochdruck und/oder von Krankheiten, die im Zusammenhang mit Bluthochdruck stehen.

Bei dem Bluthochdruck handelt es sich vorzugsweise um arterielle Hypertonie. Insbesondere kann es sich bei dem Bluthochdruck um eine therapierefraktäre Hypertonie handeln.

Die Krankheiten, die im Zusammenhang mit Bluthochdruck stehen, können ausgewählt sein aus der Gruppe umfassend Schlaganfall, Herzinfarkt, Herz- und Nierenversagen.

Die neurotoxische Substanz gemäß dem erstenErfindungsaspekt zeichnet sich vorzugsweise dadurch aus, dass sie eine Inaktivierung von spannungsabhängigen (spannungsaktivierten) Natriumkanälen (Natriumionenkanäle), insbesondere von renalen Neuronen, bevorzugt von Nervenfasern (Axonen) renaler Neuronen, inhibiert oder verlangsamt und die Substanz zur Durchführung einer renalen Denervierung vorgesehen ist.

Unter spannungsabhängigen bzw. spannungsaktivierten Natriumkanälen versteht man zelluläre Natriumkanäle, die sich in Abhängigkeit vom Membranpotential öffnen. Derzeit sind neun verschiedene Subtypen von spannungsabhängigen Natriumkanälen bekannt. Der gebräuchlichen Nomenklatur entsprechend werden sie als Naᵥ1.1 bis Naᵥ1.9 bezeichnet. Der Index v steht für *voltage-activated* (spannungsaktiviert), die erste Ziffer bezeichnet die Genfamilie (bisher nur eine bekannt), die zweite Ziffer steht für ein bekanntes Gen (zurzeit 1 bis 9, angeordnet in der Reihenfolge ihrer Entdeckung). Solche Natriumkanäle sind besonders dicht in elektrisch erregbaren Zellen, insbesondere entlang den Axonen von Neuronen (Nervenzellen), exprimiert. Dort ist ihre Aktivierung verantwortlich für die Erzeugung von Aktionspotentialen. Beim Ruhepotential sind spannungsabhängige Natriumkanäle geschlossen und aktivierbar. Bei einer Depolarisation über einen kanalspezifischen Wert erfolgt eine Konformationsänderung. Die Kanäle werden durchlässig für Natriumionen und gehen in einen offenen, d.h. aktivierten, Zustand über. Die Kanäle bleiben aber trotz anhaltender Depolarisation nicht offen, sondern werden in der Regel innerhalb weniger Millisekunden unabhängig vom Membranpotential wieder geschlossen. Dies geschieht meist durch einen im Zytoplasma liegenden Teil der Kanalproteine, die sogenannte Inaktivierungsdomäne, die sich gleich einem "Stöpsel" in den Kanal setzt und diesen verstopft, wodurch der weitere loneneinstrom limitiert wird. Diesen Zustand der Natriumkanäle bezeichnet man als geschlossen und inaktiviert. Der Übergang in den Zustand geschlossen und aktivierbar erfolgt durch eine anschließende Repolarisation und gegebenenfalls Hyperpolarisation.

Die erfindungsgemäß vorgesehene neurotoxische Substanz inhibiert (verhindert) oder verlangsamt somit ein Schließen von aktivierten, d.h. offenen, spannungsabhängigen Natriumkanälen. Dies geschieht erfindungsgemäß insbesondere dadurch, dass die neurotoxische Substanz eine für Natriumionen durchlässige Konformation der spannungsabhängigen Natriumkanäle stabilisiert.

Die vorliegende Erfindung beruht nun auf der überraschenden Erkenntnis, dass sich neurotoxische Substanzen, die eine Inaktivierung von aktivierten spannungsabhängigen Natriumkanälen inhibieren oder verlangsamen, für die Durchführung einer renalen Denervierung sowie zur Behandlung und/oder Vorbeugung von Bluthochdruck und/oder Krankheiten, die im Zusammenhang mit Bluthochdruck stehen verwenden lassen. Von Vorteil ist insbesondere, dass derartige Substanzen ein gezieltes, d.h. selektives, Adressieren von renalen Neuronen erlauben, wodurch zum einen eine umfassende Denervierung möglich ist, und zum anderen umliegende Körpergewebebereiche nicht, wenigstens jedoch nicht in nennenswertem Ausmaß, beschädigt werden. Die vorliegende Erfindung hat den weiteren Vorteil, dass sie eine vergleichsweise kostengünstige, risikoarme und einfach zu handhabende Möglichkeit zur renalen Denervierung und somit zur Behandlung und/oder Vorbeugung von Bluthochdruck und/oder Krankheiten, die im Zusammenhang mit Bluthochdruck stehen, zur Verfügung stellt.

Es versteht sich, dass der Ausdruck "neurotoxische Substanz" im Sinne der vorliegenden Erfindung nicht nur eine einzelne neurotoxische Substanz, sondern auch ein Gemisch aus verschiedenen neurotoxischen Substanzen, die jeweils ein Schließen von aktivierten spannungsabhängigen Natriumkanälen inhibieren oder verlangsamen, umfassen kann. Es versteht sich weiterhin, dass ein solches Gemisch beispielsweise alle oder jede beliebige Untergruppe der im Folgenden beispielhaft genannten neurotoxischen Substanzen enthalten kann.

Bezüglich der Bedeutung des Ausdrucks "renale Denervierung" wird auf die einleitend gemachten Erläuterungen Bezug genommen.

Der Ausdruck "proximales Ende" definiert im Sinne der vorliegenden Erfindung ein dem Rumpf eines Anwenders, in der Regel eines Arztes, zugewandtes Ende eines im Folgenden noch näher erläuterten Verabreichungsinstruments, wohingegen der Ausdruck "distales Ende" ein vom Rumpf eines Anwenders, in der Regel eines Arztes, entferntes Ende eines solchen Verabreichungsinstruments meint.

Eine erfindungsgemäße neurotoxische Substanz kann grundsätzlich zur Anwendung bei Mensch oder Tier vorgesehen sein. Bevorzugt ist eine Anwendung bei menschlichen Patienten.

### Die denervierende Wirkung der erfindungsgemäß vorgesehenen Substanz beruht auf folgendem neurotoxischen Wirkprinzip:

Die Inhibierung oder Verlangsamung der Inaktivierung der Natriumkanäle hat eine persistente, d.h. andauernde, Depolarisation renaler Neuronen zur Folge, im Zuge derer weitere spannungsabhängige lonenkanäle, insbesondere Calciumkanäle, geöffnet werden. Allgemein zeichnet sich eine persistente Depolarisierung durch einen verstärkten intrazellulär gerichteten Natrium- und Calciumionenstrom aus. Ein permanenter Natrium- und Calciumionenstrom in die Neuronen führt zur Störung des lonengleichgewichts entlang der Zellmembran, zu einem Calciumionen abhängigen Kollaps des mitochondrialen Membranpotentials, zur vermehrten Freisetzung freier Radikale und zu einem erhöhten p53-Proteinniveau, was final zum Zelltod der betroffenen Neuronen führt. Dies wiederum hat eine signifikante Verminderung des Bluthochdrucks zur Folge. Außerdem sind renale Nerven bei Bluthochdruckpatienten häufig hyperaktiv, wodurch die Wirkung der erfindungsgemäß vorgesehenen Substanz noch verstärkt wird.

Zur Erzielung der oben beschriebenen neurotoxischen Wirkung bindet die erfindungsgemäß vorgesehene Substanz, vorzugsweise selektiv, an die aktivierten spannungsabhängigen Natriumkanäle.

Grundsätzlich kann die neurotoxische Substanz an einen spannungsabhängigen Natriumkanal binden, welcher ausgewählt ist aus der Gruppe bestehend aus Naᵥ1.1, Naᵥ1.2, Naᵥ1.3, Naᵥ1.4, Naᵥ1.5, Naᵥ1.6, Naᵥ1.7, Naᵥ1.8, Naᵥ1.9 und Kombinationen davon.

Bevorzugt bindet die neurotoxische Substanz an eine als Neurotoxin Rezeptor Site 2 (neurotoxin receptor site 2) bezeichnete Position der spannungsabhängigen Natriumkanäle.

Die neurotoxische Substanz kann in neutraler, d.h. ungeladener, Form oder in Form eines Salzes vorliegen. Hinsichtlich des Salzes bestehen grundsätzlich keinerlei Limitierungen, solange hierdurch die gewünschte denervierende Wirkung der neurotoxischen Substanz nicht oder wenigstens nicht nennenswert beeinträchtigt wird. Die neurotoxische Substanz kann zum Beispiel in Form eines Chloridsalzes (Salz mit Chlorid als Gegenion), Nitratsalzes (Salz mit Nitrat als Gegenion), Sulfatsalzes (Salz mit Sulfat als Gegenion) oder in Form eines Gemisches aus diesen Salzen vorliegen.

Die neurotoxische Substanz ist ausgewählt aus der Gruppe bestehend aus Veratrumalkaloide, Sabadillalkaloide, Zygadenusalkaloide, Salze davon und Gemische davon.

Die neurotoxische Substanz ist in einer weiteren Ausführungsform ein Alkaloid mit einer sekundären Aminofunktion, vorzugsweise ausgewählt aus der Gruppe bestehend aus Jervin, Veratramin, Salze davon und Gemische davon.

In einer weiteren Ausführungsform ist die neurotoxische Substanz ein Alkaloid mit einer tertiären Aminofunktion, vorzugsweise ausgewählt aus der Gruppe bestehend aus Rubijervin, Isorubijervin, Germin, Isogermin, Pseudogermin, Veracevin (Protocevin), Cevin, Cevagenin, Zygadenin, Neosabadin, Salze davon und Gemische davon.

In einer weiteren Ausführungsform handelt es sich bei der neurotoxischen Substanz um ein Alkamin mit unbekannter Struktur, welches ausgewählt ist aus der Gruppe bestehend aus Veratrobasin, Geralbin, Protoverin, Veragenin, Salze davon und Gemisch davon.

In einer weiteren Ausführungsform handelt es sich bei der neurotoxischen Substanz um ein Esteralkaloid, welches ausgewählt ist aus der Gruppe bestehend aus Germinester, Veracevinester, Neosabadinester, Protoverinester, Salze davon und Gemische davon.

Bei der neurotoxischen Substanz kann es sich in einer weitergehenden Ausführungsform um einen Germinester handeln, welcher ausgewählt ist aus der Gruppe bestehend aus Germerin, Germidin, Isogermidin, Neogermidin, Germbudin, Neogermbudin, Desacetylgermitetrin, Germitetrin B, Germitetrin, Neogermitrin, Germitrin, Germanidin, Germanitrin, Germinitrin, Salze davon und Gemische davon.

In einer weiteren Ausführungsform handelt es sich bei der neurotoxischen Substanz um einen Veracevinester, welcher ausgewählt ist aus der Gruppe bestehend aus Cevacin, Cevadin, Veratridin, Zygacin, Vanilloylzygadenin, Veratroylzygadenin, Salze davon und Gemische davon.

Besonders bevorzugt handelt es sich bei der neurotoxischen Substanz um Veratridin (3-Veratroylveracevin). Das Alkaloid kann aus dem Samen von Sabadill (Schoenocaulon officinale) oder aus den Rhizomen des Weißen Germers (Veratrum album) gewonnen werden. Auf eine blutdrucksenkende Wirkung von Veratridin ist in der Literatur zwar vereinzelt hingewiesen worden ("Clinical Studies on Veratrum Alkaloids" von Edward Meilman und Otto Krayer: Circulation 1950; 1: 204-213 sowie US 5,288,723). Eine Verwendung von Veratridin zur Anwendung bei der renalen Denervierung ist jedoch bislang noch nicht beschrieben worden.

In einer weiteren Ausführungsform handelt es sich bei der neurotoxischen Substanz um Veratrin, d.h. ein Veratridin enthaltendes Alkaloidgemisch, wie es beispielsweise in Vertretern der Germergewächse vorkommt.

In einer weiteren Ausführungsform handelt es sich bei der neurotoxischen Substanz um einen Neosabadinester, bevorzugt um Sabadin.

In einer weiteren Ausführungsform handelt es sich bei der neurotoxischen Substanz um einen Protoverinester, welcher ausgewählt ist aus der Gruppe bestehend aus Protoveratrin A, Desacetylprotoveratrin, Protoveratrin B, Desacetylneoprotoveratrin, Escholerin, Salze davon und Gemische davon.

Die neurotoxische Substanz ist in einer weiteren Ausführungsform für eine Verabreichung als Lösung oder Suspension, insbesondere als wässrige oder organische Lösung/Suspension, vorgesehen. Im Falle einer organischen Lösung oder Suspension kann das Lösungs- oder Suspensionsmittel ausgewählt sein aus der Gruppe bestehend aus Ethanol, Aceton, Dimethysulfoxid (DMSO) und Gemische davon.

Bevorzugt ist die neurotoxischen Substanz für eine Verabreichung als Lösung vorgesehen, wobei die Lösung eine Konzentration der neurotoxischen Substanz von 0.0001 mM (mmol/l) bis 1 mM (mmol/l) aufweisen kann.

Die neurotoxische Substanz ist für eine Verabreichung mittels Injektion vorgesehen.

In einer weiteren Ausführungsform ist die neurotoxische Substanz für eine minimalinvasive Verabreichung, insbesondere für eine katheterunterstützte Verabreichung, vorgesehen.

Zur Vermeidung oder Reduzierung etwaiger Nebenwirkungen ist die neurotoxische Substanz bevorzugt für eine lokale Verabreichung vorgesehen.

Die neurotoxische Substanz ist für eine Verabreichung, vorzugsweise Injektion, in die Tunica adventitia (Tunica externa) einer renalen Arterienwand vorgesehen. Da die Tunica adventitia der renalen Arterienwand von den Nervenfasern des Sympathikus (renaler Sympathikus) innerviert wird, lässt sich auf diese Weise besonders wirkungsvoll eine renale Denervierung erzielen und das Ausmaß etwaiger unerwünschter Nebenwirkungen bleibt lokal stark begrenzt. Außerdem können geringere Dosen der neurotoxischen Substanz ausreichend sein, um die gewünschte denervierende Wirkung zu erzielen.

Weiterhin ist es erfindungsgemäß besonders bevorzugt, wenn die neurotoxische Substanz für eine lumenseitige Verabreichung, vorzugsweise Injektion, in die Tunica adventitia einer renalen Arterienwand vorgesehen ist. Unter dem Ausdruck "lumenseitige Verabreichung" soll in diesem Zusammenhang eine Verabreichung der neurotoxischen Substanz ausgehend vom Lumen einer renalen Arterie verstanden werden.

In einer weiteren bevorzugten Ausführungsform ist die neurotoxische Substanz für eine lokale Verabreichung in einer Dosis vorgesehen, welche, bezogen auf das Körpergewicht eines menschlichen oder tierischen Patienten, einer Dosis von 13.5 mg/kg Körpergewicht entspricht.

Die neurotoxische Substanz kann insbesondere für eine lokale Verabreichung in einer Dosis von 0.0005 µg/g Körpergewebe bis 13.5 µg/g Körpergewebe, insbesondere 0.0005 µg/g Körpergewebe bis 12 µg/g Körpergewebe, bevorzugt 0.05 µg/g Körpergewebe bis 10 µg/g Körpergewebe, weiter bevorzugt 0,21 µg/g Körpergewebe bis 9 µg/g Körpergewebe, besonders bevorzugt 0,42 µg/g Körpergewebe bis 8 µg/g Körpergewebe, besonders bevorzugt 5 µg/g Körpergewebe bis 7 µg/g Körpergewebe, vorgesehen sein.

In einer weiteren Ausführungsform ist die neurotoxische Substanz für die Behandlung lediglich eines Längsabschnittes einer renalen Arterie vorgesehen. Bevorzugt ist die neurotoxische Substanz für die Behandlung eines renalen Arterienlängsabschnittes vorgesehen, welcher eine Länge von 0.1 mm bis 100 mm, insbesondere 0.2 mm bis 50 mm, bevorzugt 0.5 mm bis 25 mm, besonders bevorzugt 1 mm bis 10 mm, besitzt.

Bezüglich weiterer Merkmale und Vorteile der neurotoxischen Substanz wird vollständig auf die noch folgende Beschreibung Bezug genommen.

Weiterhin wird ein Verabreichungsinstrument zur Anwendung bei der Behandlung und/oder Vorbeugung von Bluthochdruck und/oder von Krankheiten, die im Zusammenhang mit Bluthochdruck stehen, offenbart.

Das Verabreichungsinstrument zeichnet sich besonders dadurch aus, dass es eine neurotoxische Substanz gemäß der vorliegenden Erfindung enthält.

Das Verabreichungsinstrument weist vorzugsweise wenigstens eine Verabreichungseinrichtung auf.

Der Ausdruck "wenigstens eine Verabreichungseinrichtung" definiert im Sinne der vorliegenden Erfindung eine Verabreichungseinrichtung oder eine Mehrzahl von Verabreichungseinrichtungen, d.h. zwei oder mehr Verabreichungseinrichtungen.

Die wenigstens eine Verabreichungseinrichtung ist dazu ausgebildet ist, die neurotoxische Substanz gezielt in eine Tunica adventitia einer renalen Arterienwand zu verabreichen.

Zusätzlich kann die wenigstens eine Verabreichungseinrichtung dazu vorgesehen sein, eine Salzlösung, insbesondere eine natrium- und/oder calciumhaltige Salzlösung, in das zu denervierende Körpergewebe zu verabreichen. Durch die Verabreichung einer Salzlösung vor, während oder nach der Verabreichung der erfindungsgemäß vorgesehenen neurotoxischen Substanz kann die Anzahl der aktivierten spannungsabhängigen Natriumkanäle erhöht und damit die denervierende Wirkung der neurotoxischen Substanz verstärkt werden. Ist eine Verabreichung der Salzlösung während der Verabreichung der neurotoxischen Substanz erwünscht, kann die wenigstens eine Verabreichungseinrichtung beispielsweise einen mittels einer Trennwand kompartimentierten Verabreichungskanal besitzen.

Die wenigstens eine Verabreichungseinrichtung ist vorzugsweise an einem distalen Ende oder im Bereich eines distalen Endes des Verabreichungsinstruments ausgebildet.

Die wenigstens eine Verabreichungseinrichtung kann insbesondere an ihrer Außenoberfläche mit der neurotoxischen Substanz beschichtet sein.

Alternativ kann die wenigstens eine Verabreichungseinrichtung eine die neurotoxische Substanz enthaltende Beschichtung aufweisen. Die Beschichtung kann beispielsweise zusätzlich ein die Freisetzung der neurotoxischen Substanz beschleunigendes Additiv, beispielsweise in Form eines Röntgenkontrastmittels, enthalten.

In einer vorteilhaften Ausführungsform ist die wenigstens eine Verabreichungseinrichtung expandierbar ausgestaltet. Bevorzugt liegt die wenigstens eine Verabreichungseinrichtung vor einem Einführen des Verabreichungsinstruments in ein Lumen einer renalen Arterie in einem nicht expandierten Zustand vor und kann nach dem Einführen in ein Lumen einer renalen Arterie in einen expandierten Zustand überführt werden. Hierdurch lässt sich mit besonderem Vorteil ein direkter Kontakt mit einer renalen Arterienwand herstellen, wodurch hohe Freisetzungsraten der neurotoxischen Substanz realisierbar sind.

Bevorzugt handelt es sich bei der wenigstens einen Verabreichungseinrichtung um wenigstens eine Injektionseinrichtung, d.h. um eine Einrichtung, welche dazu ausgebildet ist, die neurotoxische Substanz gezielt in das zu denervierende Körpergewebe, in eine Tunica adventitia einer renalen Arterienwand, zu injizieren. Die wenigstens eine Verabreichungseinrichtung kann in Form eines die neurotoxische Substanz freisetzenden Ballons, insbesondere in Form eines Injektionsballons, einer oder mehrerer Injektionskanülen und/oder in Form einer Korb- oder Netzstruktur vorliegen. Die Korb- oder Netzstruktur kann aus massiven oder hohlen Filamenten bestehen. Im Falle einer Korb- oder Netzstruktur aus massiven Filamenten kann die Struktur mit einer die neurotoxische Substanz freisetzenden Beschichtung versehen sein. Im Fall einer aus hohlen Filamenten (Hohlfilamente) gebildeten Korb- oder Netzstruktur können die Filamente wenigstens zum Teil mit der neurotoxischen Substanz befüllt sein. Bevorzugt ist eine solche Korb- oder Netzstruktur zudem expandierbar ausgebildet und wenigstens ein Teil der Hohlfilamente nach Art einer Injektionskanüle ausgebildet, welche bei Expansion der Korb- oder Netzstruktur die renale Arterienwand, insbesondere die Tunica adventitia einer renalen Arterienwand, penetrieren.

Die wenigstens eine Verabreichungseinrichtung ist in einer weiteren Ausführungsform kühlbar ausgebildet. Beispielsweise kann es erfindungsgemäß vorgesehen sein, dass die wenigstens eine Verabreichungseinrichtung dazu ausgebildet ist, mittels einer Kühlflüssigkeit aktiv gekühlt zu werden. Insbesondere kann es im Falle einer in Form einer Korb- oder Netzstruktur aus hohlen Filamenten vorliegenden Verabreichungseinrichtung vorgesehen sein, dass wenigstens ein Teil der Hohlfilamente mit einer Kühlflüssigkeit beschickt werden kann. Durch eine Kühlung der wenigstens einen Verabreichungseinrichtung können mit besonderem Vorteil etwaige Patientenschmerzen gelindert werden.

In einer weiteren Ausführungsform weist das Verabreichungsinstrument wenigstens eine Stimulationseinrichtung auf.

Der Ausdruck "wenigstens eine Stimulationseinrichtung" definiert im Sinne der vorliegenden Erfindung eine Stimulationseinrichtung oder eine Mehrzahl von Stimulationseinrichtungen, d.h. zwei oder mehr Stimulationseinrichtungen.

Die wenigstens eine Stimulationseinrichtung ist vorzugsweise in Richtung eines proximalen Endes, insbesondere an einem proximalen Ende, des Verabreichungsinstruments ausgebildet. Die wenigstens eine Stimulationseinrichtung ist dazu ausgebildet, die spannungsabhängigen Natriumkanäle entlang von Axonen renaler Nervenfasern zu stimulieren, damit die Natriumkanäle in einen aktivierten, d.h. in einen für Natrium- und gegebenenfalls Calciumionen durchlässigen, Zustand übergehen. Hierfür kann die wenigstens eine Stimulationseinrichtung derart ausgeführt sein, dass sie eine Verabreichung einer Salzlösung, insbesondere einer natrium- und/oder calciumhaltigen Salzlösung, erlaubt. Beispielsweise kann die wenigstens eine Stimulationseinrichtung als wenigstens eine Injektionseinrichtung ausgestaltet sein, die ein Injizieren der Salzlösung in einen zu denervierenden Körpergewebebereich, insbesondere in eine renale Arterienwand, vorzugsweise in eine Tunica adventitia einer renalen Arterienwand, ermöglicht. Mit anderen Worten kann es erfindungsgemäß somit vorgesehen sein, dass das Verabreichungsinstrument wenigstens zwei Injektionseinrichtungen aufweist, wovon eine Einrichtung zum Injizieren der neurotoxischen Substanz und die andere Einrichtung zum Injizieren der Salzlösung vorgesehen ist.

Bei der in den vorherigen Absätzen erwähnten Salzlösung handelt es sich vorzugsweise um eine wässrige Salzlösung.

Alternativ kann die wenigstens eine Stimulationseinrichtung dazu ausgebildet sein, eine Stimulation, d.h. Öffnung, der spannungsabhängigen Natriumkanäle mittels Zufuhr von elektrischer Energie zu bewirken. Durch die Zufuhr von elektrischer Energie lässt sich die Anzahl an aktivierten Natriumkanälen und damit die denervierende Wirkung der neurotoxischen Substanz ebenfalls erhöhen. Auch die Zufuhr von elektrischer Energie kann grundsätzlich vor, während oder nach der Verabreichung der neurotoxischen Substanz erfolgen.

In einer vorteilhaften Ausführungsform ist die wenigstens eine Stimulationseinrichtung expandierbar ausgestaltet. Bevorzugt liegt die wenigstens eine Stimulationseinrichtung vor einem Einführen des Verabreichungsinstruments in ein Lumen einer renalen Arterie in einem nicht expandierten Zustand vor und kann nach dem Einführen in ein Lumen einer renalen Arterie in einen expandierten Zustand überführt werden. Hierdurch lässt sich mit besonderem Vorteil ein direkter Kontakt mit einer renalen Arterienwand herstellen, wodurch eine effiziente und wirksame Stimulierung möglich ist.

Die wenigstens eine Stimulationseinrichtung kann zur Stimulierung von renalen Nerven eine oder gegebenenfalls mehrere Elektroden aufweisen. Beispielsweise kann die wenigstens eine Stimulationseinrichtung in Form eines Ballons vorliegen, welcher mit einer oder mehreren Elektroden beschichtet ist. Alternativ können eine oder mehrere Elektroden in einer Wandung des Ballons, d.h. in einem Ballonmaterial, eingebunden sein. Weiterhin kann die wenigstens eine Stimulationseinrichtung in Form einer Korb- oder Netzstruktur vorliegen. Die Korb- oder Netzstruktur besteht vorzugsweise aus Filamenten, die elektrisch leitfähig sind. Die Filamente können massiv oder hohl ausgestaltet sein.

Die wenigstens eine Stimulationseinrichtung ist in einer weiteren Ausführungsform kühlbar ausgebildet. Beispielsweise kann es erfindungsgemäß vorgesehen sein, dass die wenigstens eine Stimulationseinrichtung dazu ausgebildet ist, mittels einer Kühlflüssigkeit aktiv gekühlt zu werden. Insbesondere kann es im Falle einer in Form einer Korb- oder Netzstruktur aus hohlen Filamenten vorliegenden Stimulationseinrichtung vorgesehen sein, dass wenigstens ein Teil der Hohlfilamente mit einer Kühlflüssigkeit beschickt werden kann. Durch eine Kühlung der wenigstens einen Stimulationseinrichtung können mit besonderem Vorteil etwaige Patientenschmerzen gelindert werden.

In einer weiteren Ausführungsform weist das Verabreichungsinstrument wenigstens eine Zentriereinrichtung auf.

Der Ausdruck "wenigstens eine Zentriereinrichtung" definiert im Sinne der vorliegenden Erfindung eine Zentriereinrichtung oder eine Mehrzahl von Zentriereinrichtungen, d.h. zwei oder mehr Zentriereinrichtungen.

Die wenigstens eine Zentriereinrichtung ist dazu ausgebildet, das Verabreichungsinstrument innerhalb einer menschlichen oder tierischen renalen Arterie, zu zentrieren.

In einer vorteilhaften Ausführungsform ist die wenigstens eine Zentriereinrichtung expandierbar ausgestaltet. Bevorzugt liegt die wenigstens eine Zentriereinrichtung vor einem Einführen des Verabreichungsinstruments in ein Lumen einer renalen Arterie in einem nicht expandierten Zustand vor und kann nach dem Einführen in ein Lumen einer renalen Arterie in einen expandierten Zustand überführt werden. Hierdurch lässt sich besonders effektiv eine Zentrierung des Verabreichungsinstruments innerhalb des Lumens einer renalen Arterienwand erzielen.

Die wenigstens eine Zentriereinrichtung kann beispielsweise in Form eines oder mehrerer stempelförmiger Elemente vorliegen. Das Profil des bzw. der stempelförmigen Elemente kann im Schnitt einem "T" ähneln. Alternativ kann die wenigstens eine Zentriereinrichtung in Form einer Korb- oder Netzstruktur ausgebildet sein. Die Korb- oder Netzstruktur kann aus massiven oder hohlen Filamenten bestehen. Alternativ kann die wenigstens eine Zentriereinrichtung in Form eines Ballons ausgebildet sein.

Die wenigstens eine Zentriereinrichtung ist in einer weiteren Ausführungsform kühlbar ausgebildet. Beispielsweise kann es erfindungsgemäß vorgesehen sein, dass die wenigstens eine Zentriereinrichtung dazu ausgebildet ist, mittels einer Kühlflüssigkeit aktiv gekühlt zu werden. Insbesondere kann es im Falle einer in Form einer Korb- oder Netzstruktur aus hohlen Filamenten vorliegenden Zentriereinrichtung vorgesehen sein, dass wenigstens ein Teil der Hohlfilamente mit einer Kühlflüssigkeit beschickt werden kann. Durch eine Kühlung der wenigstens einen Zentriereinrichtung können mit besonderem Vorteil etwaige Patientenschmerzen gelindert werden.

Um eine möglichst exakte in-vivo-Positionsbestimmung des Verabreichungsinstruments zu ermöglichen, weist das Verabreichungsinstrument in einer weiteren Ausführungsform wenigstens eine Markierung auf.

Der Ausdruck "wenigstens eine Markierung" definiert im Sinne der vorliegenden Erfindung eine Markierung oder eine Mehrzahl von Markierungen, d.h. zwei oder mehr Markierungen.

Erfindungsgemäß kann es beispielsweise vorgesehen sein, dass das Verabreichungsinstrument zwei Markierungen aufweist, wobei sich eine Markierung an einem distalen Ende des Verabreichungsinstruments und eine weitere Markierung zwischen wenigstens einer Stimulationseinrichtung und einem proximalen Ende des Verabreichungsinstruments befindet.

Bevorzugt handelt es sich bei der wenigstens einen Markierung um wenigstens eine radioopake Markierung.

In einer bevorzugten Ausführungsform handelt es sich bei dem Verabreichungsinstrument um einen Katheter, insbesondere um einen Ballonkatheter. Bezüglich grundsätzlich geeigneter Katheter sei beispielhaft auf die EP 0 303 757 B1 verwiesen, deren Offenbarungsgehalt bezüglich der dort beschriebenen Katheter durch ausdrückliche Bezugnahme zum Inhalt der vorliegenden Beschreibung gemacht wird.

Bezüglich weiterer Merkmale und Vorteile des Verabreichungsinstruments, insbesondere der neurotoxischen Substanz, wird vollständig auf die im Rahmen des bisherigen Erfindungsaspekts gemachten Ausführungen sowie auf die noch folgende Beschreibung Bezug genommen. Gemäß einem zweiten Aspekt betrifft die Erfindung ein medizinisches Kit zur Anwendung bei der Behandlung und/oder Vorbeugung von Bluthochdruck und/oder von Krankheiten, die im Zusammenhang mit Bluthochdruck stehen.

Das Kit weist eine neurotoxische Substanz gemäß der vorliegenden Erfindung auf. Zusätzlich weist das Kit ein Verabreichungsinstrument und eine Salzlösung auf. Zusätzlich kann das Kit ein Führungselement aufweisen.

Bei dem Führungsinstrument handelt es sich um ein Instrument, welches dazu ausgebildet ist, das Verabreichungsinstrument in ein Lumen einer renalen Arterienwand einzuführen. Bei dem Führungsinstrument handelt es sich vorzugsweise um einen Führungskatheter.

Die neurotoxische Substanz, das Verabreichungsinstrument sowie die Salzlösung liegen vorzugsweise räumlich getrennt voneinander vor.

Bezüglich weiterer Merkmale und Vorteile des medizinischen Kits, insbesondere der neurotoxischen Substanz, des Verabreichungsinstruments sowie der Salzlösung, wird vollständig auf die im Rahmen der bisherigen Beschreibung gemachten Ausführungen sowie auf die noch folgende Beschreibung Bezug genommen.

Gemäß einem dritten Aspekt wird ein Verfahren zur renalen Denervierung, vorzugsweise selektiven renalen Denervierung, bei welchem eine neurotoxische Substanz oder ein eine neurotoxische Substanz enthaltendes Arzneimittel verabreicht wird, wobei die neurotoxische Substanz eine Inaktivierung von spannungsabhängigen (spannungsaktivierten) Natriumkanälen (Natriumionenkanälen), insbesondere von renalen Neuronen, bevorzugt von Nervenfasern (Axonen) renaler Neuronen, inhibiert oder verlangsamt, offenbart.

Bevorzugt wird die neurotoxische Substanz oder das Arzneimittel als Lösung oder Suspension, insbesondere als wässrige oder organische Lösung/Suspension, verabreicht.

Vorzugsweise wird die neurotoxische Substanz oder das Arzneimittel als Lösung verabreicht, wobei die Lösung eine Konzentration der neurotoxischen Substanz oder des Arzneimittels von 0.0001 mM (mmol/l) bis 1 mM (mmol/l) aufweisen kann. Als geeignete Lösungs- oder Suspensionsmittel können Ethanol, Aceton, Dimethylsulfoxid (DMSO) oder Gemische davon verwendet werden.

Die neurotoxische Substanz oder das Arzneimittel wird bevorzugt mittels Injektion verabreicht.

Es kann grundsätzlich vorgesehen sein, dass die neurotoxische Substanz oder das Arzneimittel systemisch, bevorzugt parenteral, verabreicht wird.

Insbesondere kann die neurotoxische Substanz oder das Arzneimittel in einer systemischen Dosis von 0.001 mg/kg Körpergewicht bis 13.5 mg/kg Körpergewicht, insbesondere 0.0005 mg/kg Körpergewicht bis 12 mg/kg Körpergewicht, bevorzugt 0.05 mg/kg Körpergewicht bis 10 mg/kg Körpergewicht, weiter bevorzugt 0,21 mg/kg Körpergewicht bis 9 mg/kg Körpergewicht, besonders bevorzugt 0,42 mg/kg Körpergewicht bis 8 mg/kg Körpergewicht, besonders bevorzugt 5 mg/kg Körpergewicht bis 7 mg/kg Körpergewicht, verabreicht werden. Unter dem Ausdruck "systemische Dosis" soll eine im Rahmen einer systemischen Verabreichung eingesetzte Dosis verstanden werden.

Bevorzugt wird die neurotoxische Substanz oder das Arzneimittel minimalinvasiv, insbesondere mit Hilfe eines Katheters, verabreicht.

Bevorzugt wird die neurotoxische Substanz oder das Arzneimittel lokal verabreicht. Auf diese Weise kann das Risiko allfälliger Nebenwirkungen verringert oder sogar gänzlich vermieden werden.

Bevorzugt wird die neurotoxische Substanz oder das Arzneimittel in eine renale Arterienwand verabreicht, vorzugsweise injiziert. Hierdurch lässt sich das Risiko etwaiger unerwünschter Nebenwirkungen, wie beispielsweise Schädigungen von umliegenden Körpergewebebereichen, besonders wirkungsvoll reduzieren oder sogar vollständig vermeiden.

Erfindungsgemäß wird die neurotoxische Substanz oder das Arzneimittel in die Tunica adventitia (Tunica externa) einer renalen Arterienwand injiziert. Da die Tunica adventitia der renalen Arterienwand von den Nervenfasern des Sympathikus (renaler Sympathikus) innerviert wird, lässt sich auf diese Weise besonders wirkungsvoll eine renale Denervierung erzielen und das Ausmaß etwaiger unerwünschter Nebenwirkungen bleibt lokal stark begrenzt. Außerdem können geringere Dosen der neurotoxischen Substanz ausreichend sein, um die gewünschte denervierende Wirkung zu erzielen.

Weiter bevorzugt wird die neurotoxische Substanz oder das Arzneimittel lumenseitig in eine renale Arterienwand, vorzugsweise in die Tunica adventitia einer renalen Arterienwand, eines menschlichen oder tierischen Patienten verabreicht, vorzugsweise injiziert. Unter dem Ausdruck "lumenseitige Verabreichung" soll in diesem Zusammenhang eine Verabreichung der neurotoxischen Substanz oder des Arzneimittels ausgehend vom Lumen einer renalen Arterie verstanden werden.

Weiter bevorzugt wird die neurotoxische Substanz oder das Arzneimittel in einer lokalen Dosis verabreicht, welche, bezogen auf das Körpergewicht eines menschlichen oder tierischen Patienten, einer Dosis von 13.5 mg/kg Körpergewicht entspricht. Unter dem Ausdruck "lokale Dosis" soll eine im Rahmen einer lokalen Verabreichung eingesetzte Dosis verstanden werden.

Die neurotoxische Substanz oder das Arzneimittel kann insbesondere in einer lokalen Dosis von 0.0005 µg/g Körpergewebe bis 13.5 µg/g Körpergewebe, insbesondere 0.0005 µg/g Körpergewebe bis 12 µg/g Körpergewebe, bevorzugt 0.05 µg/g Körpergewebe bis 10 µg/g Körpergewebe, weiter bevorzugt 0,21 µg/g Körpergewebe bis 9 µg/g Körpergewebe, besonders bevorzugt 0,42 µg/g Körpergewebe bis 8 µg/g Körpergewebe, besonders bevorzugt 5 µg/g Körpergewebe bis 7 µg/g Körpergewebe, verabreicht werden.

Weiter bevorzugt wird lediglich ein Längsabschnitt einer renalen Arterie mit der neurotoxischen Substanz oder dem Arzneimittel behandelt. Bevorzugt wird ein renaler Arterienlängsabschnitt behandelt, welcher eine Länge von Länge 0.1 mm bis 100 mm, insbesondere 0.2 mm bis 50 mm, bevorzugt 0.5 mm bis 25 mm, besonders bevorzugt 1 mm bis 10 mm, besitzt.

In einer unter denervierenden Gesichtspunkten vorteilhaften Ausführungsform wird ferner eine Salzlösung, nämlich eine natrium- und/oder calciumionenhaltige, Salzlösung verabreicht.

Auf diese Weise kann eine Aktivierung von spannungsabhängigen Natriumkanälen erhöht und die denervierende Wirkung der neurotoxischen Substanz oder des Arzneimittels verstärkt werden. Bei der Salzlösung handelt es sich vorzugsweise um eine wässrige Salzlösung. Die Verabreichung der Salzlösung kann vor, während oder nach Verabreichung der neurotoxischen Substanz oder des Arzneimittels erfolgen. Die Salzlösung wird vorzugsweise in eine renale Arterienwand, insbesondere in die Tunica adventitia einer renalen Arterienwand verabreicht, vorzugsweise injiziert.

Alternativ kann die denervierende Wirkung der neurotoxischen Substanz oder des Arzneimittels erhöht werden, indem eine renale Arterienwand mittels elektrischer Energie stimuliert wird.

Die Verabreichung der neurotoxischen Substanz oder des Arzneimittels sowie gegebenenfalls eine unterstützende Stimulierung der renalen Arterienwand kann insbesondere mittels des beschriebenen Verabreichungsinstruments vorgenommen werden.

Bezüglich weiterer Merkmale und Vorteile des Verfahrens, insbesondere der neurotoxischen Substanz sowie des Verabreichungsinstruments, wird vollständig auf die in der bisherigen Beschreibung gemachten Ausführungen sowie auf die noch folgende Beschreibung Bezug genommen.

Gemäß einem vierten Aspekt wird ein Verfahren zur Behandlung und/oder Vorbeugung von Bluthochdruck und/oder von Krankheiten, die im Zusammenhang mit Bluthochdruck stehen, bei welchem eine neurotoxische Substanz oder ein eine neurotoxische Substanz enthaltendes Arzneimittel verabreicht wird, wobei die neurotoxische Substanz eine Inaktivierung von spannungsabhängigen (spannungsaktivierten) Natriumkanälen (Natriumionenkanälen), insbesondere von renalen Neuronen, bevorzugt von Nervenfasern (Axonen) renaler Neuronen, inhibiert oder verlangsamt und die neurotoxische Substanz bzw. das Arzneimittel zur Durchführung einer renalen Denervierung verwendet wird, offenbart.

Bezüglich weiterer Merkmale und Vorteile des Verfahrens, insbesondere der neurotoxischen Substanz sowie des Arzneimittels, wird vollständig auf die in der bisherigen Beschreibung gemachten Ausführungen Bezug genommen.

Weitere Merkmale und Vorteile der Erfindung ergeben sich aus der nachfolgenden Beschreibung von bevorzugten Ausführungsformen in Form von Figuren, Figurenbeschreibungen, eines Ausführungsbeispiels sowie der Unteransprüche. Dabei können einzelne Merkmale jeweils für sich alleine oder in Kombination miteinander verwirklicht sein.

### Kurze Beschreibung der Figuren

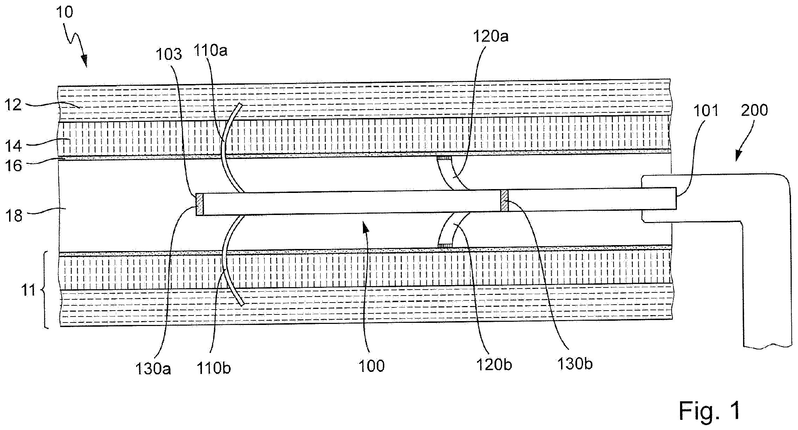

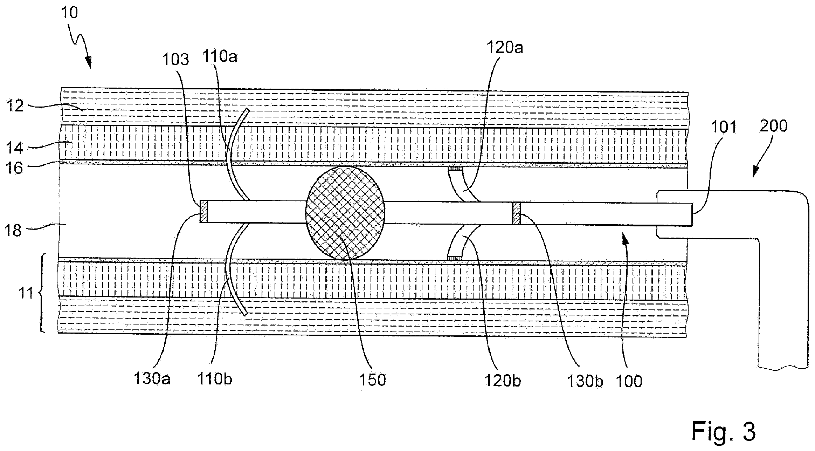

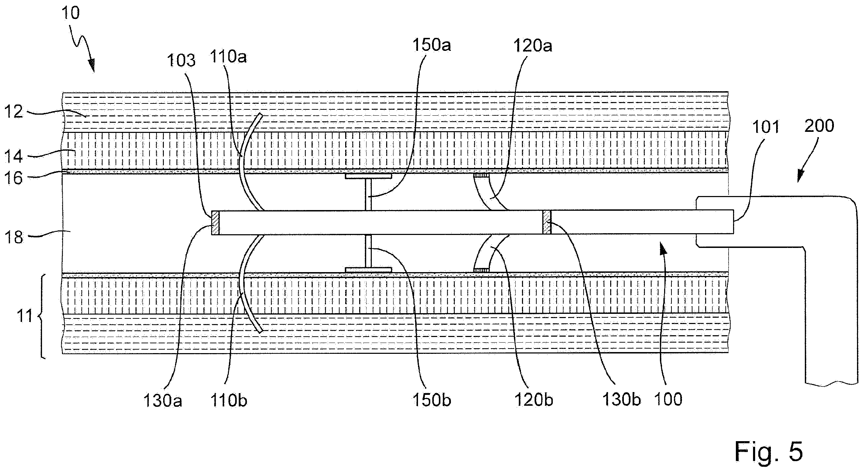

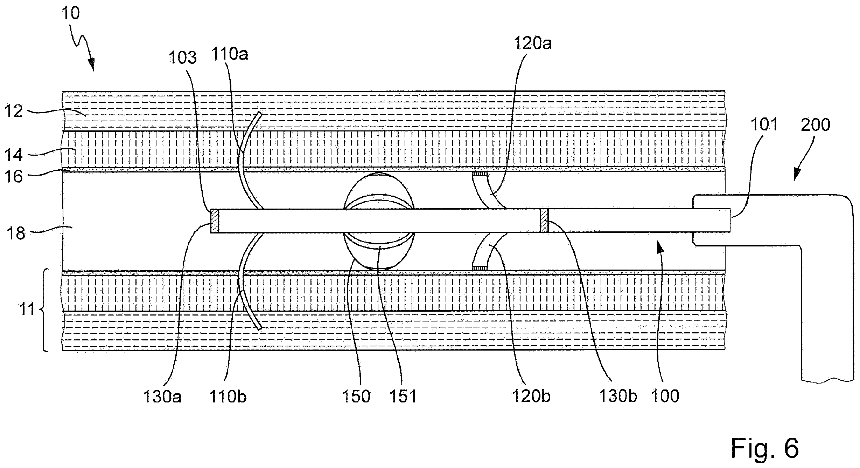

Die Figuren 1 bis 12 zeigen schematisch verschiedene Ausführungsformen eines erfindungsgemäßen Verabreichungsinstruments sowie dessen bevorzugte Verwendungsart bei der Durchführung einer renalen Denervierung.

### Detaillierte Beschreibung der Figuren

Figur 1 zeigt schematisch eine Ausführungsform eines erfindungsgemäßen Verabreichungsinstruments 100 nach Einführung in das Lumen 18 einer renalen Arterie 10. Die Wand 11 der renalen Arterie 10 setzt sich aus den drei folgenden Schichten zusammen:
Innen, das heißt dem Lumen 18 zugewandt, befindet sich die sogenannte Tunica interna 16. Die Tunica interna 16 besitzt sehr flache Zellen (Plattenepithel). Diese bilden das sogenannte Gefäßendothel. Die Tunica interna 12 steht in direktem Kontakt mit dem Blut und ist sehr glatt und sehr dünn ausgebildet. Bei der mittleren Schicht handelt es sich um sogenannte Tunica media 14, die Muskelschicht der Arterie. Sie besteht aus glatter Muskulatur, die mit feinen Bindegewebsfasern durchsetzt ist. Die äußerste Schicht ist die sogenannte Tunica adventitia 12. Sie besteht aus Bindegewebe und elastischen Fasern sowie feinen Nervenfasern des sympathischen Nervensystems, welche diese Schicht innervieren.

Das Verabreichungsinstrument besitzt ein proximales Ende 101 und ein distales Ende 103. Das Einführen des Verabreichungsinstruments kann vorteilhafterweise mittels eines Führungsinstruments 200 erfolgen. Sowohl das Verabreichungsinstrument 100 als auch das Führungsinstrument 200 sind vorzugsweise als Katheter ausgebildet.

Das Verabreichungsinstrument 100 besitzt zwei Verabreichungseinrichtungen 110a; 110b, welche dazu ausgebildet sind, eine Verabreichung einer erfindungsgemäßen neurotoxischen Substanz in die Arterienwand 11, erfindungsgemäß in die Tunica adventitia 12, zu ermöglichen. Die Verabreichungseinrichtungen 110a;110b können, wie in Figur 1 dargestellt, in Form von Injektionskanülen ausgebildet sein, welche eine selektive Injektion der neurotoxischen Substanz in die Tunica adventitia 12 erlauben. Auf diese Weise bleibt die Wirkung der neurotoxischen Substanz lokal stark begrenzt. Andere Körpergewebe als die Tunica adventitia 12 werden nicht in Mitleidenschaft gezogen, wodurch im Ergebnis eine besonders gezielte und patientenschonende renale Denervierung möglich ist.

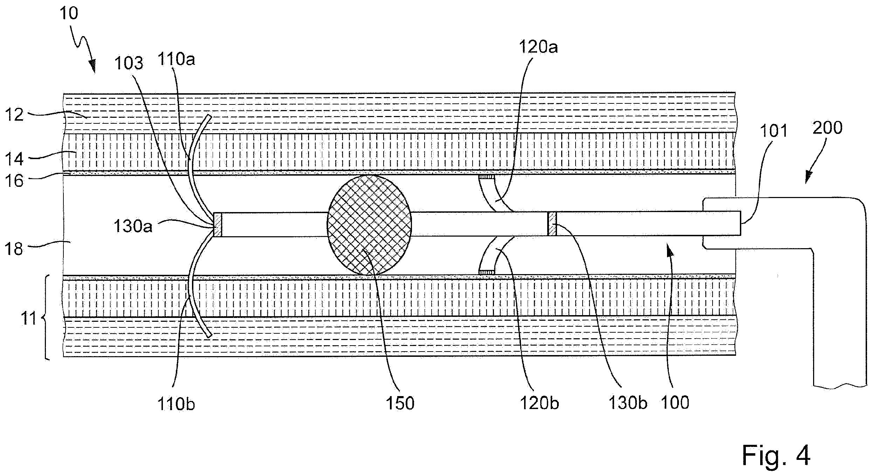

Die Verabreichungseinrichtungen 110a;110b sind vorzugsweise im Bereich des distalen Endes 103 oder am distalen Ende 103 (siehe Figur 4) ausgebildet.

Um die renale Denervierung der neurotoxischen Substanz zu verstärken, kann das Verabreichungsinstrument 100 ferner zwei Stimulationseinrichtungen 120a;120b besitzen. Die Stimulationseinrichtungen 120a;120b sind dazu ausgebildet, spannungsabhängige Natriumkanäle von renalen Nervenfasern, welche die Tunica adventitia 12 innervieren, zu stimulieren. Hierdurch gehen die Natriumkanäle in einen aktivierten, d.h. in einen für Natrium- und gegebenenfalls Calciumionen durchlässigen, Zustand über. Hierfür können die Stimulationseinrichtungen 120a;120b beispielsweise derart ausgebildet sein, dass sie eine Verabreichung, insbesondere Injektion, einer Salzlösung, bevorzugt einer wässrigen natrium- und/oder calciumhaltigen Salzlösung, erlauben. Alternativ können die Stimulationseinrichtungen 120a;120b derart ausgebildet sein, dass sie die Zufuhr von elektrischer Energie und somit eine elektrische Stimulierung der spannungsabhängigen Natriumkanäle ermöglichen.

Um eine exakte Positionsbestimmung des Verabreichungsinstruments 100 zu ermöglichen, kann es weiterhin vorgesehen sein, dass das Instrument 100 zwei radioopake Markierungen 130a;130b aufweist. Die Markierung 130a befindet sich vorzugsweise am distalen Ende 103 und die Markierung 130b bevorzugt zwischen den Stimulationseinrichtungen 120a;120b und dem proximalen Ende 103.

Das in Figur 2 schematisch dargestellte Verabreichungsinstrument 100 besitzt zusätzlich eine Zentriereinrichtung 150. Die Zentriereinrichtung 150 ist dazu ausgebildet, das Verabreichungsinstrument 100 innerhalb des Lumens 18 der renalen Arterie 10 zu zentrieren. Die Zentriereinrichtung 150 kann, wie in Figur 2 dargestellt, in Form eines Ballons ausgebildet sein. Alternativ kann die Zentriereinrichtung 150, wie in den Figuren 3 und 4 dargestellt, in Form einer expandierbaren Korb- oder Netzstruktur vorliegen. Im Zuge einer Expansion der Korb- oder Netzstruktur 150 vergrößert sich deren Durchmesser, wodurch das Verabreichungsinstrument 100 innerhalb des Lumens 18 zentriert werden kann. Alternativ kann die Zentriereinrichtung, wie in Figur 5 dargestellt, in Form von stempelförmigen Elementen 150a;150b vorliegen. Diese können ein Profil besitzen, welches im Schnitt einem "T" ähnelt. Das in Figur 6 schematisch dargestellte Verabreichungsinstrument 100 besitzt eine Zentriereinrichtung 150 in Form einer Korb- oder Netzstruktur aus hohlen Filamenten 151. Die Struktur 150 ist expandierbar ausgebildet. Aufgrund der expansionsbedingten Durchmesservergrößerung der Struktur 150 ist eine Zentrierung des Verabreichungsinstruments 100 innerhalb des Lumens 18 möglich.

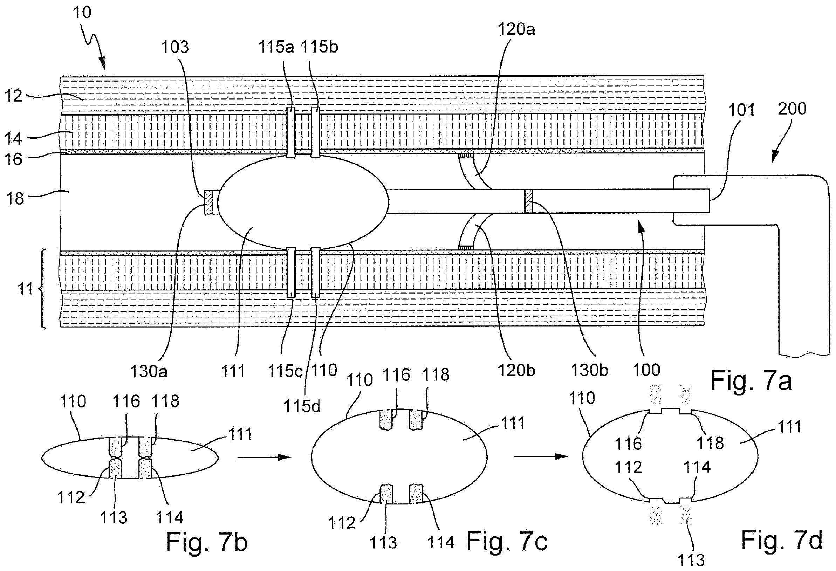

Das in Figur 7a schematisch dargestellte Verabreichungsinstrument 100 weist eine Verabreichungseinrichtung 110 in Form eines Injektionsballons sowie zwei Stimulationseinrichtungen 120a;120b auf. Der Injektionsballon 110 besitzt eine ballonförmigen Grundstruktur 111 und Injektionselemente 115a-d. Die Injektionselemente 115a-d sind dazu ausgebildet, eine erfindungsgemäße neurotoxische Substanz 113 in die Tunica adventitia 12 zu injizieren. Der Injektionsballon 110 besitzt Depots 112; 114; 116 und 118, welche jeweils mit der Substanz 113 befüllt sind. Nach Einführen des Verabreichungsinstruments 100 in das Lumen 18 der renalen Arterie 10 wird eine Expansion der ballonförmigen Grundstruktur 111 bewirkt. Im Zuge dieser Expansion wird die Grundstruktur 111 an die Tunica intima 16 angepresst, die neurotoxische Substanz 113 aus den Depots 112; 114; 116 und 118 entleert (siehe Figuren 7b bis 7d) und mittels den Injektionselementen 115a-d in die Tunica adventitia 12 injiziert.

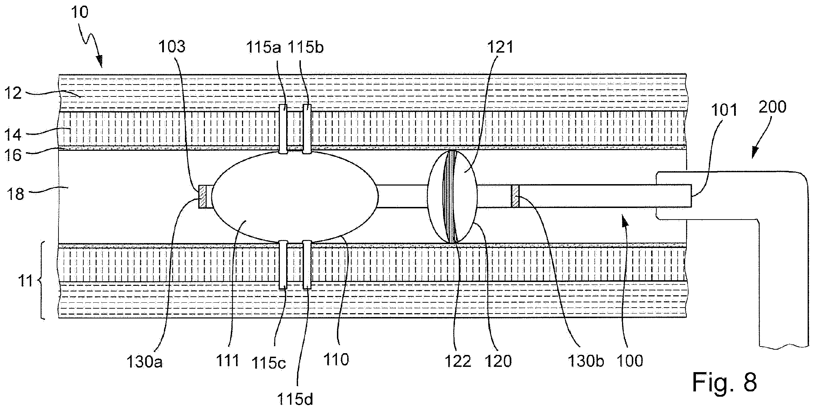

Das in Figur 8 schematisch dargestellte Verabreichungsinstrument 100 weist eine Verabreichungseinrichtung 110, wie in Figur 7a beschrieben, sowie eine Stimulationseinrichtung 120 auf. Die Stimulationseinrichtung 120 besitzt eine ballonförmige Grundstruktur 121, welche an ihrer Außenoberfläche mit einer Elektrode 122 beschichtet ist. Durch die Elektrode 122 ist eine elektrische Stimulierung von die Tunica adventitia 12 innervierenden Nervenfasern möglich.

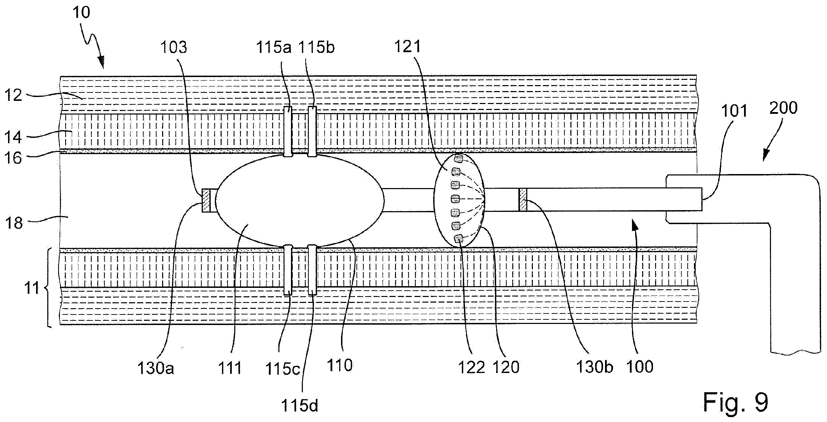

Das in Figur 9 schematisch dargestellte Verabreichungsinstrument 100 weist ebenso eine Verabreichungseinrichtung 110, wie in Figur 7a beschrieben, sowie eine Stimulationseinrichtung 120 mit einer ballonförmigen Grundstruktur 121 auf. Zur elektrischen Stimulierung von die Tunica adventitia 12 innervierenden Nerven besitzt die Stimulationseinrichtung 120 jedoch elektrisch leitende Elemente 122, welche in einem Ballonmaterial der Stimulationseinrichtung 120 eingebunden sind.

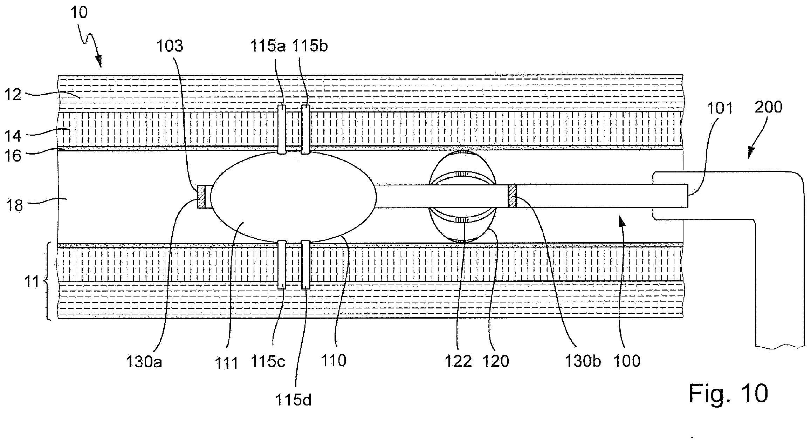

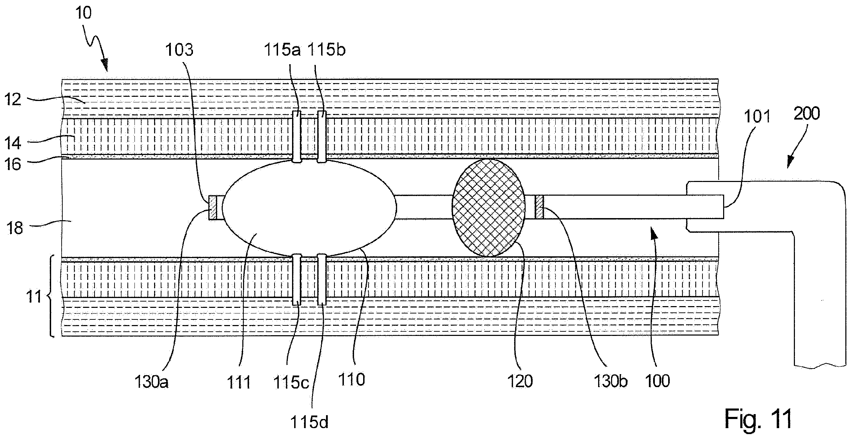

Das in Figur 10 schematisch dargestellte Verabreichungsinstrument weist eine Verabreichungseinrichtung 110, wie in Figur 7a beschrieben, sowie eine Stimulationseinrichtung 120 in Form einer expandierbaren Korb- oder Netzstruktur aus elektrisch leitfähigen, hohlen Filamenten 122 auf. Nach Einführen des Verabreichungsinstruments 100 in das Lumen 18 wird die Korb- oder Netzstruktur 120 expandiert, bis sie die Tunica intima 16 berührt. Anschließend kann eine elektrische Stimulierung der Arterienwand 11 und damit von in der Tunica adventitia verlaufenden Nervenfasern erfolgen. Alternativ kann die Stimulationseinrichtung 120, wie in Figur 11 dargestellt, in Form einer expandierbaren Korb- oder Netzstruktur aus elektrisch leitfähigen, massiven Filamenten vorliegen.

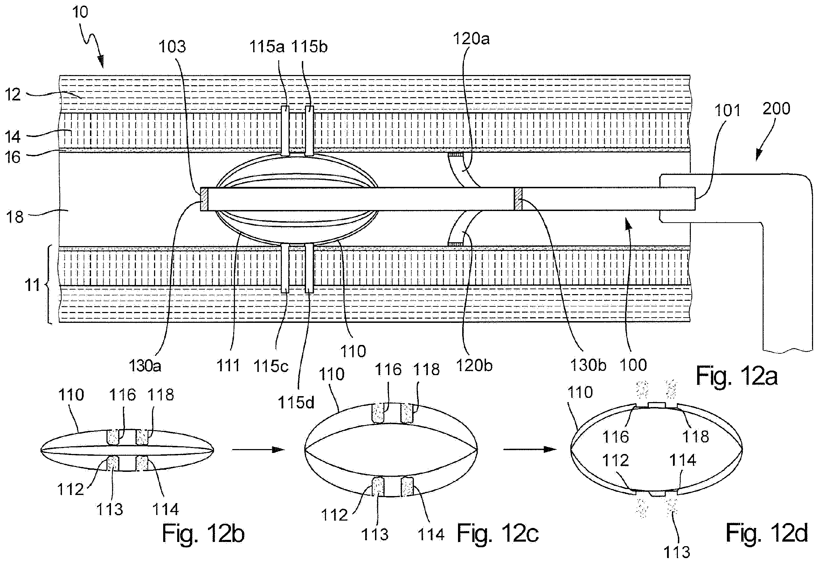

Das in Figur 12a schematisch dargestellte Verabreichungsinstrument 100 weist eine Verabreichungseinrichtung 110, welche eine korb- oder netzförmige Grundstruktur 111 aus hohlen Filamenten sowie Injektionselemente 115a-d besitzt, auf. Die Injektionselemente 115a-d sind dazu ausgebildet, eine erfindungsgemäße neurotoxische Substanz 113 in die Tunica adventitia 12 zu injizieren. Die Verabreichungseinrichtung 110 besitzt Depots 112; 114; 116 und 118, welche jeweils mit der Substanz 113 befüllt sind. Nach Einführen des Verabreichungsinstruments 100 in das Lumen 18 der renalen Arterie 10 wird eine Expansion der Grundstruktur 111 bewirkt. Im Zuge dieser Expansion wird die Grundstruktur 111 an die Tunica intima 16 angepresst, die neurotoxische Substanz 113 aus den Depots 112; 114; 116 und 118 entleert (siehe Fig. 12b bis 12d) und mittels den Injektionselementen 115a-d in die Tunica adventitia 12 injiziert. Zusätzlich weist das Verabreichungsinstrument 100 zwei Stimulationseinrichtungen 120a;120b auf.

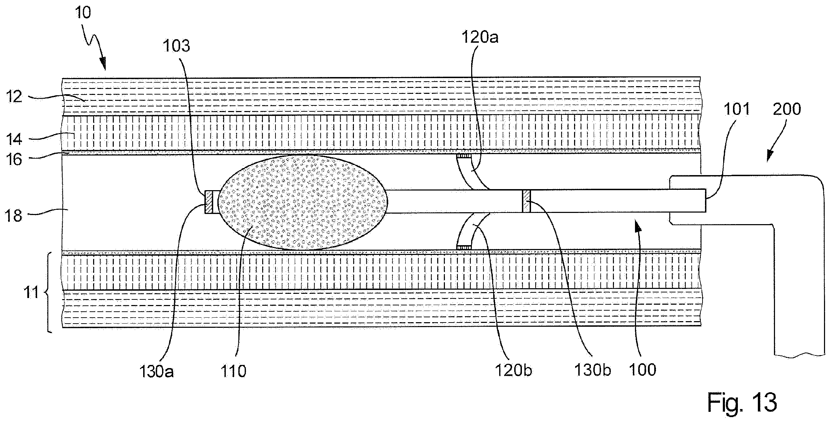

Das in Figur 13 schematisch dargestellte, nicht erfindungsgemäße Verabreichungsinstrument 100 weist eine Verabreichungseinrichtung 110 in Form eines Ballons auf, der imstande ist, eine erfindungsgemäße neurotoxische Substanz freizusetzen. Die Freisetzung der Substanz kann beispielsweise aus einer die Substanz enthaltenden Beschichtung, welche die Oberfläche des Ballons 110 bedeckt, erfolgen. Zusätzlich weist das Verabreichungsinstrument 100 zwei Stimulationseinrichtungen 120a;120b auf.

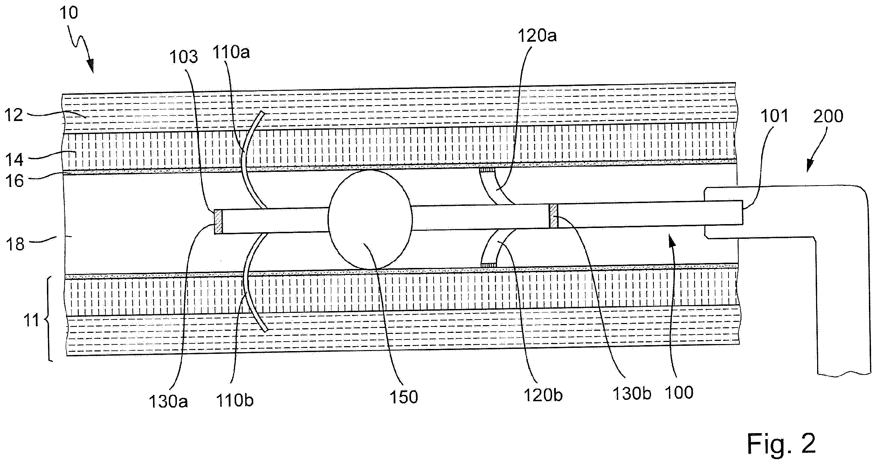

Bezüglich weiterer Merkmale und Einzelheiten zu den in den Figuren 2 bis 13 dargestellten Verabreichungsinstrumenten 100 wird vollumfänglich auf die Beschreibung zur Figur 1 Bezug genommen.

## Patentansprüche

1. Neurotoxische Substanz zur Anwendung bei der Behandlung und/oder Vorbeugung von Bluthochdruck und/oder von Krankheiten, die im Zusammenhang mit Bluthochdruck stehen, **dadurch gekennzeichnet, dass** die Substanz ausgewählt ist aus der Gruppe bestehend aus Veratrumalkaloide, Sabadillalkaloide, Zygadenusalkaloide, Salze davon und Gemische davon und die Substanz und ferner eine natrium- und/oder calciumionenhaltige Salzlösung in die Tunica adventitia einer renalen Arterienwand verabreicht, vorzugsweise injiziert, wird.

2. Neurotoxische Substanz zur Anwendung nach Anspruch 1, **dadurch gekennzeichnet, dass** es sich bei der Substanz um einen Veracevinester handelt, welcher vorzugsweise ausgewählt ist aus der Gruppe bestehend aus Cevacin, Cevadin, Veratridin, Zygacin, Vanilloylzygadenin, Veratroylzygadenin, Salze davon und Gemische davon.

3. Neurotoxische Substanz zur Anwendung nach Anspruch 1 oder 2, **dadurch gekennzeichnet, dass** es sich bei der Substanz um Veratridin oder ein Salz davon handelt.

4. Neurotoxische Substanz zur Anwendung nach einem der vorhergehenden Ansprüche, **dadurch gekennzeichnet, dass** die Substanz minimalinvasiv, insbesondere katheterunterstützt, verabreicht wird.

5. Neurotoxische Substanz zur Anwendung nach einem der vorhergehenden Ansprüche, **dadurch gekennzeichnet, dass** die Substanz lokal verabreicht wird.

6. Neurotoxische Substanz zur Anwendung nach einem der vorhergehenden Ansprüche, **dadurch gekennzeichnet, dass** die Substanz in einer Dosis von 0.0005 µg/g Körpergewebe bis 13.5 µg/g Körpergewebe, insbesondere 0.0005 µg/g Körpergewebe bis 12 µg/g Körpergewebe, bevorzugt 0.05 µg/g Körpergewebe bis 10 µg/g Körpergewebe, weiter bevorzugt 0,21 µg/g Körpergewebe bis 9 µg/g Körpergewebe, besonders bevorzugt 0,42 µg/g Körpergewebe bis 8 µg/g Körpergewebe, besonders bevorzugt 5 µg/g Körpergewebe bis 7 µg/g Körpergewebe, lokal verabreicht wird.

7. Neurotoxische Substanz zur Anwendung nach einem der vorhergehenden Ansprüche, **dadurch gekennzeichnet, dass** die Substanz für eine Behandlung eines Längsabschnittes einer renalen Arterie verwendet wird, wobei der Längsabschnitt eine Länge von 0.1 mm bis 100 mm, insbesondere 0.2 mm bis 50 mm, bevorzugt 0.5 mm bis 25 mm, besonders bevorzugt 1 mm bis 10 mm, aufweist.

8. Medizinisches Kit zur Anwendung bei der Behandlung und/oder Vorbeugung von Bluthochdruck und/oder von Krankheiten, die im Zusammenhang mit Bluthochdruck stehen, aufweisend eine neurotoxische Substanz nach einem der vorhergehenden Ansprüche, eine natrium- und/oder calciumionenhaltige Salzlösung sowie ein Verabreichungsinstrument für die neurotoxische Substanz, wobei das Verabreichungsinstrument wenigstens eine Verabreichungseinrichtung aufweist, welche dazu ausgebildet ist, die neurotoxische Substanz gezielt in eine Tunica adventitia einer renalen Arterienwand zu verabreichen.

## Claims

1. Neurotoxic substance for utilization in the treatment and/or prophylaxis of hypertension and/or of disorders related with hypertension,
**characterized in that**
the substance is selected from the group consisting of veratrum alkaloids, sabadilla alkaloids, zygadenus alkaloids, salts thereof and mixtures thereof, and the substance and further a saline solution containing sodium ions and/or calcium ions is administered to the tunica adventitia of a renal artery wall, preferably is injected.

2. Neurotoxic substance for utilization according to claim 1, **characterized in that** the substance is a veracevine ester, preferably selected from the group consisting of cevacine, cevadine, veratridine, zygacine, vanilloylzygadenine, veratroylzygadenine, salts thereof and mixtures thereof.

3. Neurotoxic substance for utilization according to claim 1 or 2, **characterized in that** the substance is veratridine or a salt thereof.

4. Neurotoxic substance for utilization according to any of the preceding claims, **characterized in that** the substance is administered via a minimally invasive administration route, in particular catheter-assisted administration route.

5. Neurotoxic substance for utilization according to any of the preceding claims, **characterized in that** the substance is administered via a local administration route.

6. Neurotoxic substance for utilization according to any of the preceding claims, **characterized in that** the substance is administered locally at a dose of 0.0005 µg/g body tissue to 13.5 µg/g body tissue, in particular 0.0005 µg/g body tissue to 12 µg/g body tissue, preferably 0.05 µg/g body tissue to 10 µg/g body tissue, further preferably 0.21 µg/g body tissue to 9 µg/g body tissue, particularly preferred 0.42 µg/g body tissue to 8 µg/g body tissue, particularly preferred 5 µg/g body tissue to 7 µg/g body tissue.

7. Neurotoxic substance for utilization according to any of the preceding claims, **characterized in that** the substance is used for a treatment of a longitudinal section of a renal artery, wherein the longitudinal section has a length of 0.1 mm to 100 mm, in particular 0.2 mm to 50 mm, preferably 0.5 mm to 25 mm, particularly preferred 1 mm to 10 mm.

8. Medical kit for utilization in the treatment and/or prophylaxis of hypertension and/or of disorders related with hypertension, comprising a neurotoxic substance according to any of the preceding claims, a saline solution containing sodium ions and/or calcium ions, and an administration instrument for the neurotoxic substance, wherein the administration instrument includes at least one administration device which is configured to administer the neurotoxic substance specifically into a tunica adventitia of a renal artery wall.

## Revendications

1. Substance neurotoxique pour une application dans le traitement et/ou la prophylaxie de l'hypertension artérielle et/ou de maladies qui sont en rapport avec l'hypertension artérielle, **caractérisée en ce que** la substance est choisie dans le groupe constitué par les alcaloïdes de *Veratrum,* les alcaloïdes de sabadilla, les alcaloïdes de *Zygadenus,* sels correspondants et mélanges correspondants et la substance et également une solution saline contenant des ions sodium et/ou des ions calcium est administrée dans la tunica adventitia d'une paroi artérielle rénale, de préférence est injectée.

2. Substance neurotoxique pour une application selon la revendication 1, **caractérisée en ce que** la substance est un ester de véracévine, qui est de préférence choisi dans le groupe constitué par la cévacine, la cévadine, la vératridine, la zygacine, la vaniloylzygadénine, la veratroylzygadénine, des sels correspondants et des mélanges correspondants.

3. Substance neurotoxique pour une application selon la revendication 1 ou 2, **caractérisée en ce que** la substance est la vératridine ou un sel correspondant.

4. Substance neurotoxique pour une application selon l'une quelconque des revendications précédentes, **caractérisée en ce que** la substance est administrée de manière mini-invasive, en particulier à l'aide d'un cathéter.

5. Substance neurotoxique pour une application selon l'une quelconque des revendications précédentes, **caractérisée en ce que** la substance est administrée localement.

6. Substance neurotoxique pour une application selon l'une quelconque des revendications précédentes, **caractérisée en ce que** la substance est administrée localement en une dose de 0,0005 µg/g de tissu corporel à 13,5 µg/g de tissu corporel, en particulier de 0,0005 µg/g de tissu corporel à 12 µg/g de tissu corporel, préférablement de 0,05 µg/g de tissu corporel à 10 µg/g de tissu corporel, plus préférablement de 0,21 µg/g de tissu corporel à 9 µg/g de tissu corporel, particulièrement préférablement de 0,42 µg/g de tissu corporel à 8 µg/g de tissu corporel, particulièrement préférablement de 5 µg/g de tissu corporel à 7 µg/g de tissu corporel.

7. Substance neurotoxique pour une application selon l'une quelconque des revendications précédentes, **caractérisée en ce que** la substance est utilisée pour un traitement d'une section longitudinale d'une artère rénale, la section longitudinale présentant une longueur de 0,1 mm à 100 mm, en particulier de 0,2 mm à 50 mm, préférablement de 0,5 mm à 25 mm, particulièrement préférablement de 1 mm à 10 mm.

8. Kit médical pour une application dans le traitement et/ou la prophylaxie de l'hypertension artérielle et/ou de maladies qui sont en rapport avec l'hypertension artérielle, présentant une substance neurotoxique selon l'une quelconque des revendications précédentes, une solution saline contenant des ions sodium et/ou des ions calcium ainsi qu'un instrument d'administration pour la substance neurotoxique, l'instrument d'administration présentant au moins un dispositif d'administration, qui est conçu pour administrer la substance neurotoxique de manière ciblée dans une tunica adventitia d'une paroi artérielle rénale.
